# EUROPÄISCHE PATENTSCHRIFT

(11) **EP 0 711 839 B1**
(45) Veröffentlichungstag und Bekanntmachung des Hinweises auf die Patenterteilung: **04.06.2003**
(21) Anmeldenummer: 95116178.5
(22) Anmeldetag: 13.10.1995
(51) Int. Cl.: C12Q 1/56, G01N 33/86, G01N 33/96

(54) **Kalibrator zur Verwendung in Testverfahren zum Nachweis eines defekten Gerinnungsfaktors V**
Calibrator for use in testing method to detect a defective clothing factor V
Calibrateur pour usage dans une méthode d'épreuve pour détecter un agent de coagulation V défectueux

(30) Priorität: 09.11.1994 DE 4439756
(43) Veröffentlichungstag der Anmeldung: 15.05.1996
(73) Patentinhaber: Dade Behring Marburg GmbH, 35001 Marburg (DE)
(72) Erfinder: Kraus, Michael, Dr., D-35041 Marburg (DE)

(56) Entgegenhaltungen:
- WO-A-94/17415
- NATURE, Bd. 369, - 5.Mai 1994 LONDON UK, Seiten 64-67, XP 000563812 R.M. BERTINA ET AL. 'Mutation in blood coagulation factor V associated with resistance to activated protein C.'
- DRUG RESEARCH, Bd. 44, Nr. 6, - 1.Juni 1994 BASEL CH, Seiten 793-797, XP 000562757 H.E. KARGES ET AL. 'Activity of coagulation and fibrinolysis parameters in animals.'
- THROMBOSIS RESEARCH, Bd. 80, Nr. 3, - 1995 NEW YORK NY USA, Seiten 255-264, XP 000564298 M. KRAUS ET AL. 'Coagualtion assay with improved specificity to factor V mutants insensitive to activated protein C.'

## Beschreibung

Die Erfindung bezieht sich auf ein Plasma, das als Kalibrator in Gerinnungstests verwendet werden kann, die den Abbau von Faktor V durch aktiviertes Protein C erfassen, die Herstellung eines solchen Plasmas sowie seine Verwendung.

Das Gerinnungssystem im Blut sorgt zum einen für die Aufrechterhaltung des Blutstroms zu dem zu versorgenden Gewebe, zum anderen reagiert es auf Verletzungen mit einem Wundverschluß und sorgt damit für die Erhaltung der Integrität des Organismus. Bei der Aktivierung der Gerinnung entsteht über ein kaskaden-ähnliches System sich schrittweise aktivierender Proteasen letztlich die aktive Protease Thrombin. Die anfänglich nur sehr langsame Thrombinbildung wird durch Thrombin selbst beschleunigt, in dem es die Kofaktoren Faktor V und Faktor VIII durch proteolytische Spaltung aktiviert. Diese aktivierten Kofaktoren bilden mit den Proteasen Faktor Xa bzw. IXa aktive Enzym/Kofaktor-Komplexe auf Phospholipidoberflächen, deren Aktivität um den Faktor ca. 1000 höher ist als der singulären Proteasen. Durch diese positive Rückkopplung kommt es quasi explosionsartig zur Entstehung großer Mengen an Thrombin. Thrombin setzt Fibrinogen zu Fibrin um, das im Normalfall zum Wundverschluß und zur Wundheilung führt. Um eine lebensbedrohende Ausweitung der Gerinnung zu verhindern, die zu einem Verschluß des Gefäßsystems im Körper, also zu Thrombosen führen würden, müssen sowohl die aktive Protease als auch die Nachlieferung der Protease unterbunden werden. Aktive Proteasen werden im Körper durch Protease-Inhibitoren durch die Ausbildung kovalenter Komplexe neutralisiert. Die Unterbrechung des Nachschubs wird durch Thrombin selbst initiiert. Thrombin bindet hierzu an das Membranprotein Thrombomodulin und setzt das Proenzym Protein C zur aktiven Protease Protein Ca (APC) um APC seinerseits bildet mit dem Kofaktor Protein S einen Komplex, der die aktiven Kofaktoren Faktor Vllla und Faktor Va proteolytisch spaltet und dadurch inaktiviert. APC unterbricht somit die starke Stimulierung durch diese Kofaktoren.

Dieses oben beschriebene Protein C/Protein S-System stellt einen wichtigen antikoagulatorischen Mechanismus dar Dies wird dadurch bestätigt, daß Personen mit erblichen oder erworbenen Mängeln oder Defekten an Protein C oder Protein S mit hoher Wahrscheinlichkeit Thrombosen, insbesondere rezidivierende venöse Thrombosen, erleiden (Esmon, C.T. TCM 2 : 214-219,1992).

Neben Protein C und Protein S können weitere Faktoren die Aktivität des Systems beeinflussen. So der von Willebrand Faktor und Faktor IXa (Rick, M.E. et al. J. Lab. Clin. Med. 115: 415-421, 1990), die Faktor VIIIa vor proteolytischem Abbau schützen können.

Erworbene Störungen können auch auf die Entstehung von Lupus anticoagulants zurückgehen. Dies sind gegen Phospholipide gerichtete Antikörper, die die zur Funktion notwendige Anbindung der Protease/Kofaktor Komplexe an Phospholipid-Oberflächen stören (Amer, L. et al. Thromb. Res. 57: 247-258, 1990).

Schließlich wurde in jüngster Zeit eine Mutante des Faktors V beschrieben, der nicht mehr oder zumindest nur sehr schlecht durch APC inaktiviert werden kann (Bertina, R.M. et al. Nature 369: 64-67, 1994).

Diese Störungen des Protein C/Protein S Systems, mit Ausnahme des Protein C selbst, können diagnostisch relativ einfach durch eine Modifikation eines üblichen Screening Verfahren, der aktivierten partiellen Thromboplastin Zeit (APTT) erfaßt werden (Amer, L. et al., 1990), das im folgenden mit APC-Zeit (APCT) bezeichnet wird. Zur Bestimmung der APTT wird eine Plasmaprobe mit einem gleichen Volumenanteil eines Reagenzes in Kontakt gebracht, das einen Oberflächenaktivator, etwa Silica, Kaolin oder Glas und Phospholipide enthält. Dieses Gemisch wird wenige Minuten bei +37 °C inkubiert. Während dieser Zeit werden die nicht Calcium-abhängigen Faktoren des Gerinnungssystems (Faktor XII, Faktor XI und Faktor IX) aktiviert. Nach Zugabe von Calciumionen wird die restliche Gerinnungskaskade aktiviert und es bildet sich Thrombin. Die entstandene Thrombinmenge wird dann entweder durch die Umsetzung des natürlichen Substrats Fibrinogen zu einem Gerinnsel oder durch die Freisetzung eines Chromophor aus einem chromogenen Substrat bestimmt. In der Modifikation dieser APTT zur APCT wird gleichzeitig mit den Calciumionen aktiviertes Protein C zugegeben. Da, wie oben beschrieben, APC die Cofaktoren Vllla und Va zerstört, kommt es zu einer Verzögerung der Thrombinbildung, die abhängig ist von der Funktionalität des Protein C/Protein S Systems.

Die Auswertung der Ergebnisse erfolgt in der Gerinnungsdiagnostik üblicherweise an einer Referenzkurve in Prozent der Norm. Ein Plasmapool aus normalen Blutspendern definiert dabei den 100 %-Wert, während die weiteren Kalibrationspunkte üblicherweise durch Verdünnungen dieses Plasmapools mit physiologischer Kochsalzlösung erstellt werden. Diese Vorgehensweise ist aber für die Kalibrierung der APCT nicht geeignet, da durch die Verdünnung des Plasmas die Gerinnungszeit verlängert wird (siehe Beispiel 1); in Tests zur Funktionalität des Protein C/Protein S Systems ist aber eine pathologische Probe durch eine weniger starke Verlängerung der Gerinnungszeit gekennzeichnet, da in ihr die Gerinnungsaktivität nur ungenügend gedämpft wird.

Weiterhin ist es in der Gerinnungsdiagnostik üblich, zur Kalibrierung ein normales Plasma (100 %-Wert) mit einem Plasma zu mischen, aus dem der zu untersuchende Faktor entfernt wurde. Auch dieses Verfahren ist für die Kalibrierung der APCT nicht geeignet, wie Beispiel 1 zeigt. Wie die Untersuchungen von Bertina et al. (1994) gezeigt haben, ist eine wesentliche Ursache für eine ungenügend lange APCT eine Mutation im Faktor V. Ein Faktor V-Mangelplasma kann nicht verwendet werden, da dieses Plasma sowohl in der APTT wie auch in der APCT aufgrund des Faktorenmangels praktisch ungerinnbar ist.

In WO 94/17415 wird eine Plasmapräparation beschrieben, bei der ein humanes Faktor V-Mangelplasma mit Faktor V vom Rind versetzt wird. Die Verwendung der Mischung dieser Plasmapräparation mit Humanplasma als Kontrollplasma wird erwähnt. Ein Nachteil dieses Kontrollplasmas ist jedoch die aufwendige Herstellungsweise die u.a. Schritte wie die Entfernung der "antikoagulatorischen Aktivität von Faktor V" durch Faktor V-lmmunadsorption und die Herstellung von Rinder-Faktor V mitumfaßt.

Theoretisch wäre es auch denkbar, Kalibratoren aus dem Blut Betroffener zu gewinnen. Ein solches Verfahren ist aber aus ethischen und technischen Gründen nicht praktikabel.

Ähnliche Überlegungen gelten auch für andere Gerinnungsstörungen, die auf mutierte Gerinnungsfaktoren zurückzuführen sind.

Der Erfindung lag somit die Aufgabe zugrunde, einen Kalibrator zu finden, der unabhängig von Blutspenden der Betroffenen hergestellt werden kann.

Die Lösung dieser Aufgabe erfolgt durch die Bereitstellung der in den Patentansprüchen beschriebenen Ausführungsformen.

Überraschenderweise wurde gefunden, daß der Zusatz bestimmter tierischer Plasmen zu humanem Plasma konzentrationsabhängig zu einer Verkürzung der Gerinnungszeit in einer APCT führt (siehe Beispiel 2). Dieser Befund ist um so mehr überraschend, als bekannt ist, daß viele Tierplasmen einen deutlich höheren Faktor V Gehalt aufweisen als humanes Plasma (Karges et al., Drug Res. 44: 793-797, 1994). Bei Zusatz von nicht-humanem Plasma zu einem Plasma mit mutierten Faktor V wäre somit mit einer Neutralisierung der verkürzten APCT zu rechnen, da der mutierte Faktor V durch den zugegebenen Faktor V überspielt werden müßte. Aber genau der gegenteilige Effekt war zu beobachten (siehe Beispiel 3). Die gefundene Wirkung beruht wahrscheinlich darauf, daß das im Test eingesetzte humane aktivierte Protein C nicht in der Lage ist, nicht-humane Gerinnungsfaktoren proteolytisch zu spalten.

Bisher war dem Fachmann allgemein bekannt, daß Faktor V aus Tierplasma sich in den bekannten pro-koagulatorischen Testverfahren wie humaner Faktor V verhält. Es war also zu erwarten, daß tierischer Faktor V auch in dem APCT Test sich entsprechend dem humanen Faktor V verhalten würde. Weitere Untersuchungen ergaben überraschenderweise, daß die untersuchten Plasmen verschiedener Tierspecies in unterschiedlichem Ausmaß zur Verwendung in einem erfindungsgemäßen Kalibrator geeignet waren. Durch die Bereitstellung der erfindungsgemäßen Lehre ist die Eignung durch einen einfachen Versuch zu ermitteln, wobei in der APCT Bestimmung geeignete Plasmen durch eine Verkürzung der Gerinnungszeit gekennzeichnet sind. Als besonders geeignet haben sich Plasmen, bzw. Faktor V aus Kaninchen und Hund erwiesen, während beispielsweise Pferdeplasma weniger geeignet ist.

Die tierischen Faktor V Präparate können bevorzugterweise in Form eines nicht-humanen Plasmas verwendet werden. Besonders geeignet sind Kaninchen- und Hunde-Plasma.

Anstatt des nicht-humanen Plasmas kann auch eine Faktor V enthaltene Fraktion eines Hunde- oder Kaninchenplasmas oder gentechnisch hergestellter reiner Hunde- oder Kaninchen-Faktor V verwendet werden.

Die Konzentration des nicht-humanen, Faktor V-haltigen Zusatzes im humanen Citrat-Plasma wird so gewählt, daß die Reaktivität des Kalibrators sich deutlich von der ohne Zusatz unterscheidet. Bevorzugt ist ein Kalibrator, wonach der Zusatz so gewählt wird, daß sich die Gerinnungszeit mindestens um 20 %, bevorzugterweise jedoch um 50 % von der Gerinnnungszeit mit nicht versetztem, humanen Citrat-Plasma unterscheidet.

Durch den Zusatz von solchen nicht-humanen Plasmen bzw. von Fraktionen, die Faktor V vom Kaninchen oder Hund enthalten zu einem humanen Plasma, können somit Kalibratoren hergestellt werden, die einen Defekt des Faktor V in funktionellen Tests zur Überprüfung der Funktionalität des Faktor V und/oder des Protein C/Protein S Systems simulieren.

Die Kalibratoren können auch für Gerinnungsteste verwendet werden, in denen die Abbaubarkeit des Faktor Va durch aktiviertes Protein C überprüft wird. Femer können die Kalibratoren auch in Gerinnungstesten, die entstehendes Thrombin über die Bildung eines Fibringerinnsels oder/und über den Umsatz eines synthetischen Thrombinsubstrats chromogen bestimmen, verwendet werden.

Die folgenden Beispiele sollen die Erfindung erläutern.

Verwendete Abkürzungen
- APCT :: aktivierte Protein C Zeit
- APTT: aktivierte, partielle Thromboplastin Zeit
- DBA: Diethylbarbiturat-Acetat
- F.V-Def.: humanes Plasma mit einer Mutation im Faktor V
- SHP: Standard-Human-Plasma

### Beispiel 1

### Herstellung eines Kalibrators durch Verdünnung eines Plasmapools normaler Blutspender

Die Bestimmung der Gerinnungszeit erfolgte an einem mechanischen Koagulometer nach Schnitger & Gross (Fa. Amelungen). Alle Reagenzien waren von der Fa. Behringwerke AG. Als Plasmapool normaler Blutspender wurde Standard-Human-Plasma verwendet.

Die Bestimmung der APTT erfolgt nach Vorschrift: 1 Abfüllung Pathromtin® für 5 ml, ein Phospholipidgemisch aus humaner Plazenta, wurde in 5 ml Kaolinsuspension als Oberflächenaktivator gelöst. Die Calciumchloridlösung (25 mM) wurde vor Gebrauch auf +37 °C erwärmt.

In ein Meßröhrchen wurden nacheinander pipettiert
100 µl Pathromtin®
100 µl Plasmaprobe.

Anschließend wurde bei +37 °C für 2 Minuten inkubiert und durch Zugabe von 100 µl Calciumchlorid-Lösung die Gerinnungszeit gestartet. Gleichzeitig wurde eine eingebaute Stoppuhr eingeschalten und die Zeit erfaßt, bis ein Gerinnsel detektiert wurde.

Die Bestimmung der APCT erfolgte mit den APC-Sensitivitätsreagenzien der Fa. Behringwerke. Wie bei der APTT wurde das Aktivatorreagenz hergestellt. Das Startreagenz aus Calciumchlorid und aktiviertem Protein C wurde in 5 ml destilliertem Wasser gelöst und vor Gebrauch auf +37 °C erwärmt.

In ein Meßröhrchen wurden nacheinander pipettiert
100 µl Pathromtin®
100 µl Plasmaprobe.

Anschließend wurde bei +37 °C für 3 Minuten inkubiert und durch Zugabe von 100 µl Startreagenz die Gerinnungszeit gestartet. Gleichzeitig wurde eine eingebaute Stoppuhr eingeschalten und die Zeit erfaßt, bis ein Gerinnsel detektiert wurde.

SHP wurde in verschiedenen Konzentrationen physiologischer Kochsalzlösung bzw. in DBA-Puffer (Fa. Behringwerke) verdünnt und die APTT und APCT bestimmt. In Tabelle 1 ist zu ersehen, daß unabhängig vom verwendeten Medium sowohl APTT als auch APCT mit steigender Plasmaverdünnung zunehmen. Dieses Verfahren ist somit nicht geeignet, einen Kalibrator herzustellen, der gegenüber einem normalen Plasma eine verkürzte APCT aufweist.

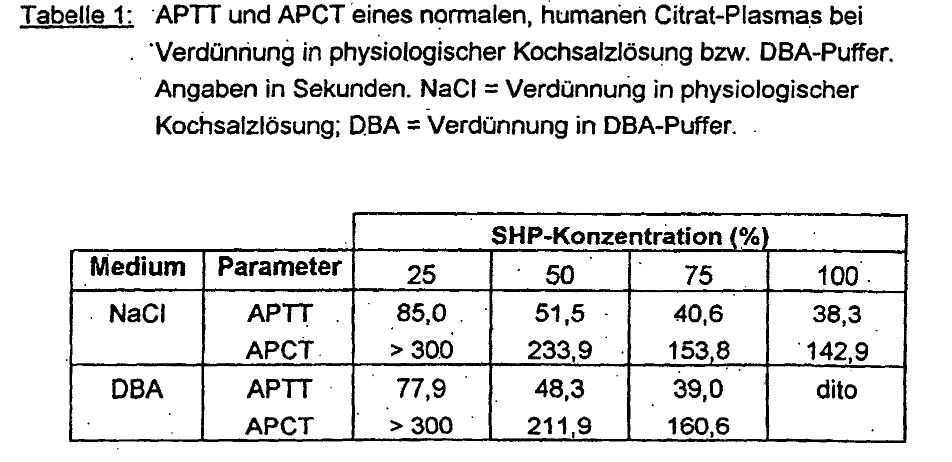

### Beispiel 2

### Herstellung eines Kalibrators durch Zusatz von nicht-humanem Plasma zu humanem Plasma

Die Bestimmungen der Gerinnungszeit erfolgten wie unter Beispiel 1 aufgeführt :

In Tabelle 2 sind die erhaltenen Gerinnungszeiten (Mittelwerte aus Doppelbestimmungen) zusammengefaßt, die erhalten wurden, indem normales humanes Citratplasma (SHP) mit Citratplasma vom Kaninchen bzw. vom Hund gemischt wurde. Es ist zu erkennen, daß mit steigendem Anteil des nicht-humanen Plasmas die APCT des Gemisches verkürzt ist. Gegenüber dem Plasma vom Hund ist der Effekt von Kaninchen-Plasma deutlich ausgeprägter. Durch den Zusatz von bereits geringen Mengen von nicht-humanem Plasma zu humanem Plasma läßt sich somit ein Defekt im Protein C/Protein S System in der APCT simulieren. Dieses Verfahren ist somit geeignet, entsprechend definierte Kalibratoren herzustellen.

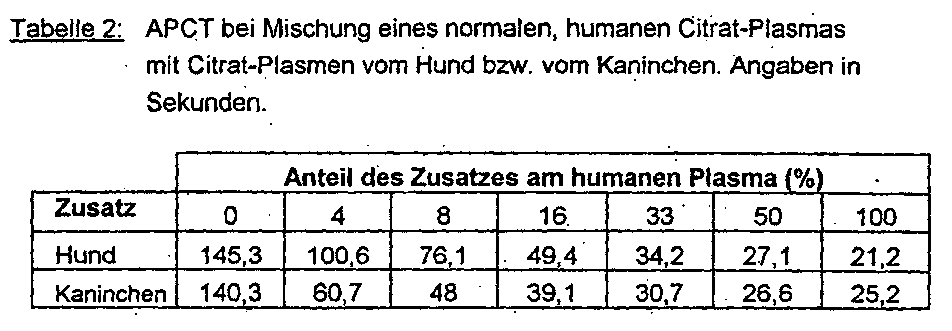

### Beispiel 3

### Effekt der Zugabe von humanem und nicht-humanem Plasma zu einem humanem Plasma mit einem Defekt im Faktor V Gen

Die Bestimmungen der Gerinnungszeit erfolgten wie unter Beispiel 1 aufgeführt.

Ein normales Plasma (SHP; siehe Beispiel 1) und Citrat-Plasma vom Kaninchen wurden einem humanen Plasma mit einem nur schwer durch APC inaktivierbaren Faktor V in verschiedenen Konzentrationen beigemischt und der Effekt auf die APCT verfolgt (Tabelle 3). Erwartungsgemäß führte ein Überschuß an normalem, humanen Plasma zu einer Neutralisierung des nicht abbaubaren Faktor V, so daß in der APCT längere Gerinnungszeiten zu beobachten waren. Im Gegensatz dazu führten bereits geringe Zugaben von Kaninchenplasma zu einer noch ausgeprägteren Verkürzung der APCT, wie dies in Beispiel 2 auch mit normalem humanen Plasma gezeigt wurde.

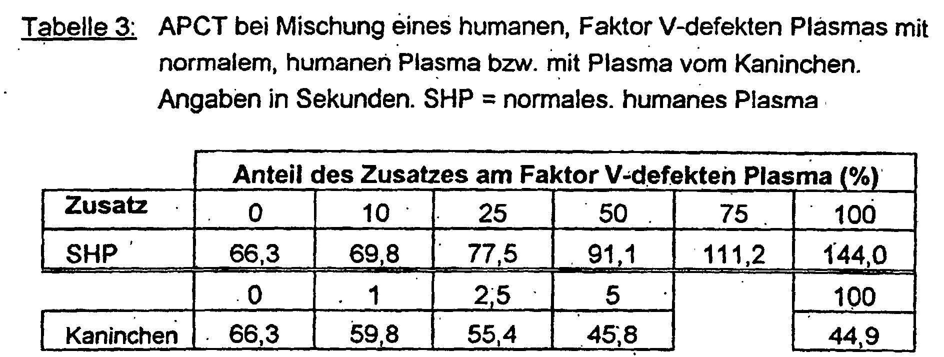

## Patentansprüche

1. Kalibrator zur Verwendung in Testverfahren zum Nachweis eines defekten Gerinnungsfaktors V, wobei der Kalibrator nicht-humanes Plasma und humanes Plasma enthält.

2. Kalibrator nach Anspruch 1, wobei Plasma einer Spezies verwendet wird, die in einem F.V- bzw. F.Va-abhängigen Testverfahren eine Änderung des Ergebnisses gegenüber einem normalen humanen Plasma zeigt.

3. Kalibrator gemäß Anspruch 1, wobei das nicht-humane Plasma in APC abhängigen Testen eine Verkürzung der Gerinnungszeit bewirkt

4. Kalibrator nach Anspruch 1 bis 3, wobei das nicht-humane Plasma ein Citrat-Plasma ist.

5. Kalibrator nach Anspruch 1 bis 3, wobei das humane Plasma ein humanes Citrat-Plasma ist, besonders bevorzugt ein nach gängigen Verfahren stabilisiertes, humanes Citrat-Plasma.

6. Kalibrator nach Anspruch 5, wobei die Konzentration des nicht-humanen, Plasmas im humanen Citrat-Plasma so gewählt wird, daß sich die Gerinnungszeit mindestens um 20 %, bevorzugterweise jedoch um 50 % von der Gerinnungszeit mit nicht versetztem, humanen Citrat-Plasma unterscheidet.

7. Kalibrator nach Anspruch 1, wobei der Kalibrator nicht-humanes Plasma vom Hund oder Kaninchen und humanes Plasma enthält.

8. Kalibrator zur Verwendung in Testverfahren zum Nachweis eines defekten Gerinnungsfaktors V, wobei der Kalibrator humanes Plasma und eine Faktor V enthaltende Fraktion eines Hunde- oder Kaninchenplasmas oder gentechnisch hergestellten reinen Hunde- oder Kaninchen-Faktor V enthält.

9. Verwendung des Kalibrators nach Anspruch 1 bis 8 für Gerinnungsteste, die die Funktionalität des Protein C/Protein S Systems überprüfen.

10. Verwendung des Kalibrators nach Anspruch 1 bis 8 für Gerinnungsteste, in denen die Abbaubarkeit des Faktor Va durch aktiviertes Protein C überprüft wird.

11. Verwendung nach Anspruch 9, wobei aktiviertes Protein C exogen zugefügt wird.

12. Verwendung nach Anspruch 9 wobei das Protein C der Probe aktiviert wird.

13. Verwendung des Kalibrators nach Anspruch 1 bis 8 in Gerinnungstesten, die entstehendes Thrombin über die Bildung eines Fibringerinnsels und/oder über. den Umsatz eines synthetischen Thrombinsubstrats chromogen bestimmen.

14. Verfahren zur Herstellung eines Kalibrators nach mindestens einem Anspruch 1 bis 8 für Verwendung in Gerinnungsassays, wobei ein humanes Plasma durch Zugabe von nicht-humanem Plasma oder Faktor V enthaltende Fraktionen aus Hunde- oder Kaninchen-Plasma aufgestockt wird.

15. Verwendung von nicht-humanem Plasma, insbesondere vom Hund oder Kaninchen, oder eine Faktor V enthaltende Fraktion aus Hunde- oder Kaninchenplasma oder eines gentechnisch hergestellten reinen Hunde- oder Kaninchen-Faktors V als Zusatz zu humanem Plasma zur Herstellung eines Kalibrators für Gerinnungsteste.

## Claims

1. A calibrator for use in test methods for detecting a defective coagulation factor V, said calibrator containing non-human plasma and human plasma.

2. The calibrator as claimed in claim 1, wherein plasma is used from a species which, in an F.V-dependent or F.Va-dependent test method, gives a different result from that obtained with a normal human plasma.

3. The calibrator as claimed in claim 1, wherein the non-human plasma brings about a reduction in the clotting time in APC-dependent tests.

4. The calibrator as claimed in claims 1 to 3, wherein the non-human plasma is a citrate plasma.

5. The calibrator as claimed in claims 1 to 3, wherein the human plasma is a human citrate plasma, particularly preferably a human citrate plasma which is stabilized in accordance with current methods.

6. The calibrator as claimed in claim 5, wherein the concentration of the non-human plasma in the human citrate plasma is chosen such that the clotting time differs by at least 20%, preferably, however, by 50%, from the clotting time obtained with untreated human citrate plasma.

7. The calibrator as claimed in claim 1, said calibrator containing non-human plasma from the dog or the rabbit, and human plasma.

8. A calibrator for use in test methods for detecting a defective coagulation factor V, said calibrator containing human plasma and a factor V-containing fraction of a dog or rabbit plasma or genetically engineered pure dog or rabbit factor V.

9. The use of the calibrator as claimed in claims 1 to 8, for coagulation tests which examine the functional efficiency of the protein C/protein S system.

10. The use of the calibrator as claimed in claims 1 to 8, for coagulation tests in which the ability of activated protein C to degrade factor Va is examined.

11. The use as claimed in claim 9, wherein activated protein C is added exogenously.

12. The use as claimed in claim 9, wherein the protein C of the sample is activated.

13. The use of the calibrator as claimed in claims 1 to 8 in coagulation tests which determine the quantity of thrombin produced by way of the formation of a fibrin clot and/or chromogenically by way of the conversion of a synthetic thrombin substrate.

14. A method for preparing a calibrator as claimed in at least one of claims 1 to 8 for use in coagulation assays, wherein a human plasma is supplemented by adding non-human plasma or factor V-containing fractions from dog or rabbit plasma.

15. The use of non-human plasma, in particular from the dog or the rabbit, or a factor V-containing fraction from dog or rabbit plasma or of a genetically engineered pure dog or rabbit factor V as an addition to human plasma for preparing a calibrator for coagulation tests.

## Revendications

1. Calibrateur à utiliser dans une méthode d'épreuve pour détecter un agent de coagulation V défectueux, où le calibrateur contient du plasma non humain et du plasma humain.

2. Calibrateur selon la revendication 1, où on utilise le plasma d'une espèce qui présente dans une méthode d'épreuve dépendant du F.V ou du F.Va, une modification des résultats vis-à-vis d'un plasma humain normal.

3. Calibrateur selon la revendication 1, où le plasma non humain agit dans des tests dépendant de l'APC, par un raccourcissement de la durée de coagulation.

4. Calibrateur selon les revendications 1 à 3, où le plasma non humain est un plasma citrate.

5. Calibrateur selon les revendications 1 à 3, où le plasma humain est un plasma citrate humain, de manière particulièrement préférée un plasma citrate humain, stabilisé selon un procédé courant.

6. Calibrateur selon la revendication 5, où la concentration du plasma non humain dans le plasma citrate humain, est choisie de sorte que la durée de coagulation se différencie d'au moins 20%, de manière préférée de 50% de la durée de coagulation du plasma citrate humain non supplémenté.

7. Calibrateur selon la revendication 1, où le calibrateur contient du plasma non humain de chien ou de lapin et du plasma humain.

8. Calibrateur à utiliser dans une méthode d'épreuve pour détecter un agent de coagulation V défectueux, où le calibrateur contient du plasma humain et une fraction contenant le facteur V d'un plasma de chien ou de lapin ou un facteur V de chien ou de lapin, pur, préparé par génie génétique.

9. Utilisation du calibrateur selon les revendications 1 à 8, pour des tests de coagulation, qui étudient la fonctionnalité du système protéine C/protéine S.

10. Utilisation du calibrateur selon les revendications 1 à 8, pour des tests de coagulation, dans lesquels la dégradabilité du facteur Va est étudiée par la protéine C activée.

11. Utilisation selon la revendication 9, où la protéine C activée esf introduite de manière exogène.

12. Utilisation selon la revendication 9, où la protéine C de l'échantillon est activée.

13. Utilisation du calibrateur selon les revendications 1 à 8, pour des tests de coagulation, qui déterminent de manière chromogène, la thrombine formée par la formation d'un caillot de fibrine et/ou par l'addition d'un substrat synthétique de la thrombine.

14. Procédé de préparation d'un calibrateur selon au moins l'une des revendications 1 à 8, à utiliser dans des dosages de coagulation, où un plasma humain est augmenté par addition de plasma non humain ou de fractions contenant du facteur V de plasma de chien ou de lapin.

15. Utilisation de plasma non humain, en particulier de chien ou de lapin, ou d'une fraction contenant du facteur V de plasma de chien ou de lapin ou d'un facteur V de chien ou de lapin, pur, préparé par génie génétique, comme additif à du plasma humain pour préparer un calibrateur pour des tests de coagulation.
